(19) Europäisches Patentamt European Patent Office Office européen des brevets

(11) **EP 3 648 695 B1**

(12) **EUROPEAN PATENT SPECIFICATION**

(45) Date of publication and mention
of the grant of the patent:
**16.06.2021 Bulletin 2021/24**

(21) Application number: **18739808.6**

(22) Date of filing: **03.07.2018**

(51) Int Cl.:
***A61B 18/18*** *(2006.01)*

(86) International application number:
**PCT/EP2018/067996**

(87) International publication number:
**WO 2019/007984 (10.01.2019 Gazette 2019/02)**

(54) **APPARATUS FOR THERMALLY TREATING LIGAMENTS**

VORRICHTUNG ZUR WÄRMEBEHANDLUNG VON BÄNDERN

APPAREIL POUR LE TRAITEMENT THERMIQUE DES LIGAMENTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.07.2017 GB 201710793**

(43) Date of publication of application:
**13.05.2020 Bulletin 2020/20**

(73) Proprietor: **Creo Medical Limited
Chepstow, Monmouthshire NP16 5UH (GB)**

(72) Inventors:
• **HANCOCK, Christopher Paul
Bath Bath and
North East Somerset BA1 4LN (GB)**
• **BURN, Patrick
Chepstow
Monmouthshire NP16 5UH (GB)**
• **GEOGHEGAN, Leif
Chepstow
Monmouthire NP16 5UH (GB)**

(74) Representative: **Mewburn Ellis LLP
Aurora Building
Counterslip
Bristol BS1 6BX (GB)**

(56) References cited:
**US-A1- 2004 133 254    US-A1- 2008 234 574
US-A1- 2010 145 328    US-A1- 2012 172 865
US-A1- 2012 191 072    US-A1- 2013 282 002
US-A1- 2015 045 786    US-A1- 2017 105 799
US-A1- 2017 118 806**

## Description

TECHNICAL FIELD

The invention relates to apparatus for carrying out ligament tightening through heat induced denaturation of collagen structures.

BACKGROUND TO THE INVENTION

Ligaments are plastic cable-like structures made up of interwoven collagen threads that connect bone to bone to form joints. With age or violent injury, the ligaments in a joint may be damaged, e.g. torn or stretched. This can cause pain and instability in the joint.

One way of repairing stretched ligaments is by heating them. The heat causes the ligaments to shrink and tighten. A technique of non-contact heating known as thermal capsulorrhaphy for treating the capsule ligaments in the shoulder has been developed based on this concept. In thermal capsulorrhaphy, a probe is inserted invasively into the shoulder joint. The tip of the probe is arranged to emit radiofrequency (RF) electromagnetic energy which thermally excites molecules in its immediate vicinity. The probe tip itself is not hot.

One problem associated with thermal capsulorrhaphy, identified in US 2002/0095199, is that the temperature induced by the probe in the ligaments is high enough to cause irreversible injury to nerves. Of particular concern is the axillary nerve, which passes directly beneath the inferior glenohumeral ligaments. If the ligament is heated in close proximity to the nerve, there is a risk of permanent injury. Since the actual path taken by the nerve can vary from human to human, it is not possible to designate a region of the ligament that is always safe for treatment.

US 2002/0095199 tackles this problem by configuring the probe to emit nerve stimulation pulses (e.g. having a so-called coagulation RF waveform) before the thermal ligament treatment is activated. If the nerve stimulation pulses stimulate a nerve (which may be visually observed), it may be understood that the region around the probe is not safe for thermal ligament treatment. The probe may be moved around, e.g. by the surgeon, until a suitable treatment zone is found.

It is also known to deliver microwave energy to effect a change in ligaments through the controlled contraction of collagen. For example, US 6,461,353 discloses an orthopaedic apparatus having a trocar with a deflectable distal end. An electrode is positioned at the distal end to deliver microwave energy at a treatment site. Furthermore, reference is made to document US 2015/045786 A1.

SUMMARY OF THE INVENTION

The invention is defined in the appended set of claims. At its most general, the present invention provides an electrosurgical apparatus for tightening ligaments using microwave energy in which a detector is used to obtain information about the conditions in or properties of the treatment zone that permits energy to delivered in a manner that causes the necessary thermal effects to target tissue (e.g. ligaments, tendons or the like) without unwanted thermal side effects, e.g. collateral thermal damage to nerve tissue or surrounding skin or fascia structures.

According to the invention, there is provided an electrosurgical apparatus for ligament tightening, the apparatus comprising: an electrosurgical generator arranged to generate and output microwave electromagnetic (EM) energy; a probe connected to the electrosurgical generator, the probe comprising: a flexible shaft containing a coaxial transmission line for conveying the microwave EM energy; and an applicator at a distal end of the flexible shaft, the applicator having an energy delivery structure arranged to receive the microwave EM from the coaxial transmission line and emit the received microwave EM energy into a treatment zone adjacent to the applicator; a detector arranged to monitor a property of the treatment zone; and a controller arranged to control an energy delivery profile of the microwave EM energy delivered to the probe based on information obtained by the detector. With this apparatus, energy can be delivered in a precise manner to the target tissue. The apparatus can ensure that collateral damage to surrounding tissue is avoided, e.g. by monitoring the treatment site to detect tissue type or sense a level of energy delivery in order to control the energy delivery profile accordingly.

In one example, the detector may comprise a temperature sensor, e.g. a thermocouple or the like. The temperature sensor may be mounted at the distal end of the applicator, e.g. to detect a temperature in the treatment zone. The detector may comprise an imaging device, e.g. to provide visual feedback of the treatment zone. The imaging device may effectively be a temperature sensor, e.g. to provide a visual indication of differing temperatures within the treatment zone. The imaging device may be operate using optical radiation, e.g. in the visible spectrum or infrared. It may comprise an optical fibre bundle extending along the flexible shaft to convey optical radiation to and from the treatment zone. In other examples, the imaging device can use other modalities, e.g. ultrasound or the like.

The detector may comprise a power sensing module arranged to detect a forward power signal travelling from the electrosurgical generator to the probe and a reflected power signal reflected back from the probe, and wherein the controller is arranged to process the detected forward and reflected power signals to obtain information indicative of a type of body tissue in the treatment zone. Accordingly, the controller may be arranged to use the output of the detector to automatically detect a suitable treatment zone. For example, the invention may measure the dielectric properties of material (body tissue) in a treatment zone located at the distal end of a probe. A control apparatus may be arranged to automatically control treatment based on the measurement. In one embodiment, the measurement may be made by detecting a signal reflected from the distal end of the probe and comparing the reflected signal with a forward signal in order to determine attenuation and/or phase constants of the material in the treatment zone. The forward signal may then be adjusted based on this comparison, i.e. to control the energy delivered to the treatment zone.

The invention may provide the facility to detect changes in the treatment zone. For example, the apparatus may react automatically if an unsuitable zone is detected during treatment. The apparatus may thus provide a responsive and sensitive apparatus, which can reduce the risk of injury, e.g. to nerves/nerve tissue.

The controller may operate automatically based on the obtained information, which may reduce the time that nerve tissue is be exposed to potentially harmful radiation. An appropriately modulated microwave energy delivery profile can effectively cause near-instant heating effects in a treatment zone, with minimal heating effects elsewhere. So, by operating the controller automatically based on the obtained information, efficient ligament tightening can be performed, while preventing damage to nerve tissue (e.g. nerve tissue in the treatment zone, and/or near the treatment zone).

Herein, type of body tissue encompasses body tissue containing nerve tissue, and body tissue not containing nerve tissue. Where ligament tightening is performed, type of body tissue preferably includes body tissue containing substantially only ligament tissue. In some embodiments, type of body tissue may also refer to the type of ligament tissue (e.g. knee, shoulder, ankle, etc.).

The controller may be arranged to determine from the detected forward and reflected power signals either: (i) complex impedance, or (ii) attenuation and/or phase constants of the type of body tissue in the treatment zone, the information indicative of the type of body tissue in the treatment zone being a result of determining the complex impedance or the attenuation and/or phase constants. The controller may include a memory storing reference data, and a microprocessor arranged to execute software commands to compare the information indicative of type of body tissue in the treatment zone with the reference data, and control the energy delivery profile based on the comparison.

The apparatus may include a cooling mechanism for removing thermal energy from the treatment zone. The cooling mechanism may include a means for bringing a cooling medium (e.g. fluid, such as water or saline) into thermal contact with the treatment zone, e.g. via the applicator. In one example, the probe may include a fluid feed conduit extending through the flexible shaft. The cooling mechanism may comprise an actuator for delivering coolant through the fluid feed conduit to the treatment zone.

The cooling mechanism may be used to provide a linear distribution of the desired temperature effect. For example, the apparatus may be arranged to provide a balance of cooling at a surface of the treatment zone with heating within the treatment zone to create an even temperature profile. A temperature in the range 60°C-70°C may be an optimum temperature for causing shrinkage of collagen in tendons or ligaments. Over 80°C the collagen will complete lose all its structure, so any thermal region above such temperature will have an undesired outcome.

The energy delivery profile may be arranged to have a limited maximum power level, e.g. equal to or less than 15 W. There is a risk that the heating at higher power can lead to reduced tensile strength of the tissue. It may be beneficial to use several applications of energy on different regions of the tissue to get the desired shrinkage without lowering the tensile strength of the device. A way to potentially to improve speed during procedures there could be more than one applicators where the distances could be adjusted to get the desired tissue affect.

The apparatus may be particularly suited for minimally invasive surgery. For example, the apparatus may include a surgical scoping device (e.g. an endoscope, gastroscope, bronchoscope, laparoscope, or the like) having a steerable instrument cord with an instrument channel extending therethrough. The probe may be dimensioned to be insertable through the instrument channel to reach the treatment zone.

The energy delivery profile may be either: a measurement energy delivery profile, or a therapeutic energy delivery profile. A power magnitude of the therapeutic energy delivery profile may be larger (e.g. an order of magnitude larger) than a power magnitude of the measurement energy delivery profile.

The controller may be arranged to detect the presence of nerve tissue in the treatment zone from the comparison. Microwave energy at the measurement power magnitude may be used in a measurement mode for safely locating a treatment zone not containing nerve tissue. Microwave energy at the therapeutic power magnitude may then be used in a therapeutic mode to thermally treat ligament tissue once a treatment zone not containing nerve tissue has been located using the measurement mode. In other words, the measurement mode may be used to identify a safe/suitable treatment zone, before the therapeutic mode is used/activated. The controller may be configured to control the energy delivery profile accordingly. For example, the controller may be arranged to select the therapeutic energy delivery profile when it is determined that nerve tissue is not present in the treatment zone.

A power magnitude of the measurement energy delivery profile may be selected so as to be sufficient to detect dielectric properties, e.g. the presence or absence of nerve tissue, but insufficient to cause significant heating effects in the treatment zone, and hence insufficient to damage nerve tissue. The power magnitude of the therapeutic energy delivery profile may be selected so as to be sufficient to cause heating effects in ligament tissue, i.e. sufficient to produce the therapeutic effect of ligament tightening. The therapeutic power magnitude may be one or more orders of magnitude higher than the measurement power magnitude, and may be sufficient to quickly heat the tissue to a temperature greater than 55°C, e.g. in the range 70°C to 80°C. The measurement power magnitude may be 10 mW (10 dBm) or less. Accordingly, it may be possible to keep the temperature in nerve tissue below (preferably substantially below) 55°C by using the measurement mode. It may therefore be possible to prevent permanent nerve damage from occurring (where permanent nerve damage has been shown to occur at temperatures exceeding 55°C). The therapeutic power magnitude may be 10 W or more (but no more than 15 W, as discussed above).

It may be preferable to deliver radiation in the measurement mode according to a continuous wave (CW) energy delivery profile for examining the reflected energy signal, from which the dielectric properties of the tissue in the treatment zone may be determined. The therapeutic mode may then use a pulsed energy delivery profile consisting of one or more pulse(s), to produce the desired therapeutic effect. In some embodiments, a single short-lived pulse may be sufficient to cause the desired near-instant heating effects in the ligament tissue of the treatment zone.

The energy delivery structure may comprise any suitable emitter for radiating an electric field with the received microwave EM energy. For example, the energy delivery structure may comprise any of: a travelling wave slotted radiator; a microstrip antenna; and an open waveguide. The energy delivery structure may be arranged to conform to a treatment zone on the human or animal body. For example, the applicator may comprise an inflatable portion arranged to expand to extend the energy delivery structure into the treatment zone. In one example, the probe may comprise a hook portion for retain a portion of tissue against the energy delivery structure.

As briefly discussed above, once a suitable treatment zone has been detected, the apparatus may be arranged to deliver power to the antenna at the therapeutic power magnitude, using an energy delivery profile selected from a plurality of energy delivery profiles. Each delivery profile may be associated with a respective ligament type. The controller may be arranged to control the variable attenuator and/or signal modulation device to deliver the forward power signal according to a delivery profile. The delivery profile may be automatically selectable by the controller according to the obtained information indicative of tissue type in the treatment zone. Alternatively or additionally, the apparatus may include a user interface connected to the controller for permitting user selection of an appropriate/desired delivery profile, e.g. dependant on an area of the body (knee, shoulder, etc.) being treated.

Furthermore, when the suitable treatment zone has been detected, the apparatus may include an impedance adjuster connected on the generator, the impedance adjuster having an adjustable complex impedance that is controllable by the controller based on the microwave detection signal to match the detected impedance of the body tissue in the treatment zone. Moreover, the forward and reflected power signals are used to monitor the power delivered to the treatment zone, so that maximum energy transfer to ligament (non-nerve) tissue is achieved. By dynamically adjusting the impedance as the therapeutic ligament tightening is carried out, it may also be possible to ensure maximum power delivery, even as the dielectric properties of collagen change through heating. In other words, as the reflection coefficient of collagen changes as it is heated, the present apparatus detects this change to maximise power delivery. The change may also be monitored, in order to monitor the progress of the tightening treatment. The dosage of microwave energy delivered into the tissue may be accurately quantified.

The output power may have a frequency in the range 1 GHz to 300 GHz. The following frequency bands in particular may be used: 2.4 GHz to 2.5 GHz, 5.725 GHz to 5.875 GHz, 14 GHz to 14.5 GHz, 24 GHz to 24.25 GHz, 30 GHz to 32 GHz, and 45 GHz to 47 GHz. Even more specifically, the following spot frequencies may be considered: 2.45 GHz, 5.8 GHz, 14.5 GHz, 24 GHz, 31 GHz, 45 GHz and 61.25 GHz. At these high frequencies, the depth of penetration of the radiation (which relates to the size of the treatment zone) is small, which both aids control of the location of the treatment zone and the ability to measure clearly the dielectric properties of material in the treatment zone. There may also be some benefit of the microwave energy dehydrating the tissue which will also assist with the target tissue shrinkage.

The antenna may comprise a travelling wave slotted radiator in an emitting region at its distal end.

In another aspect, the invention may be used in a method of thermally treating ligament tissue, the method comprising: locating an antenna at a treatment zone; emitting a microwave frequency electromagnetic field from the antenna into the treatment zone to cause heating of biological tissue in the treatment zone; detecting a forward power signal delivered to the antenna and a reflected power signal reflected back from the antenna; determining from the detected forward and reflected power signals a change in the dielectric properties of biological tissue in the treatment zone; and controlling a magnitude of the forward power signal based on the determined change in dielectric properties.

In a yet further aspect, the invention may be used in a method of thermally treating ligament tissue, the method comprising: locating an antenna at a treatment zone; emitting a microwave frequency electromagnetic field at a measurement power level from the antenna into the treatment zone; detecting a forward power signal delivered to the antenna and a reflected power signal reflected back from the antenna; determining from the detected forward and reflected power signals the presence or absence of nerve tissue in the treatment zone; and if nerve tissue is determined to be absent from the treatment zone, emitting the microwave frequency electromagnetic field at a therapeutic power level from the antenna into the treatment zone, the therapeutic power level having a magnitude greater than the measurement power level.

The apparatus of the invention may be used to treat shoulder ligaments (e.g. in thermal capsulorrhaphy), ankle ligaments (i.e. to treat ankle instability), and knee ligaments (e.g. to treat collateral ligament injuries).

BRIEF DESCRIPTION OF THE DRAWINGS

Examples of the invention are described in detail below with reference to the accompanying drawings, in which:

Fig. 1 is an overall schematic apparatus diagram of electrosurgical apparatus according to a first embodiment of the invention;
Fig. 2 is a schematic diagram of electrosurgical apparatus according to a second embodiment of the invention;
Fig. 3 is a schematic circuit diagram of an impedance adjuster and a microwave signal detector used in embodiments of the invention;
Fig. 4 is a schematic circuit diagram of another example of an impedance adjuster suitable for use in embodiments of the invention;
Fig. 5 is a schematic circuit diagram of yet another example of an impedance adjuster suitable for use in embodiments of the invention;
Fig. 6 is a schematic diagram of the complete microwave energy delivery structure treated as a distributed element circuit;
Fig. 7 is a schematic apparatus diagram of electrosurgical apparatus according to a third embodiment of the invention having a separate measurement channel;
Fig. 8 is a schematic apparatus diagram of another electrosurgical apparatus according to the third embodiment of the invention, having a separate measurement channel and having a means for tuning on the generator;
Fig. 9 is a schematic view of a general probe structure suitable for use with the invention;
Figs. 10A and 10B are schematic top and cross-sectional side views of a first example probe structure;
Fig. 11 is a schematic cross-section side view of a second example probe structure;
Figs. 12A and 12B are schematic cross-sectional side and top views of a third example probe structure;
Fig. 13 is a schematic side view of a fourth example probe structure;
Figs. 14A and 14B are schematic views of a fifth example probe structure in an undeployed and deployed configuration respectively;
Fig. 15 is a schematic view of a sixth example probe structure; and
Fig. 16 is a schematic view of a seventh example probe structure.

DETAILED DESCRIPTION; FURTHER OPTIONS AND PREFERENCES

In overview, the ligament treatment apparatus provides a means for generating high frequency microwave power at a source which is coupled to an energy delivery structure located at the distal end of a ligament tightening probe, the energy delivery structure being adapted to launch a focussed electromagnetic field into a small delicate ligament tissue structure to cause a virtually instant local temperature rise, which may enable efficient ligament or muscle tightening to be performed.

Moreover, the apparatus may include a means for measuring dielectric properties of material (body tissue) into which the electromagnetic field is launched. By providing a sensitive measurement apparatus, heating effects can be confined to treatment zones containing substantially only target tissue (e.g. ligament tissue), and damage to nerve tissue is prevented.

With suitably formed probe structures, the invention can be used to treat eye muscle ligaments, knee ligaments, ankle ligaments and/or shoulder ligaments, for example.

Some embodiments discussed below incorporate tissue type identification techniques, however the invention need not be limited to such techniques. These techniques are capable of characterizing the type of tissue at the treatment zone according to dielectric properties using a measurement mode, in order to determine which of nerve tissue and ligament tissue is present in the treatment zone. For example, the magnitude of microwave power delivered to the antenna may be adjusted accordingly, e.g. substantially increased when no nerve tissue is detected, so as to deliver energy suitable for tightening ligaments in a therapeutic mode. Heating effects in the treatment zone from the delivery of microwave radiation to nerve tissue can therefore be substantially reduced, preventing nerve damage from occurring.

Some embodiments discussed below also incorporate dynamic tissue matching techniques to ensure maximum energy delivery into tissue over a range of impedances that can vary from less than 10 $\Omega$ to greater than 100 k$\Omega$, when a suitable treatment zone (e.g. a treatment zone which does not contain nerve tissue) is been detected.

In other embodiments, the ligament treatment probe may include a temperature sensor or other transducer providing an output indicative of temperature at the treatment zone. The delivery of microwave energy may be controlled based on the detected temperature.

*Overall apparatus and generator configuration*

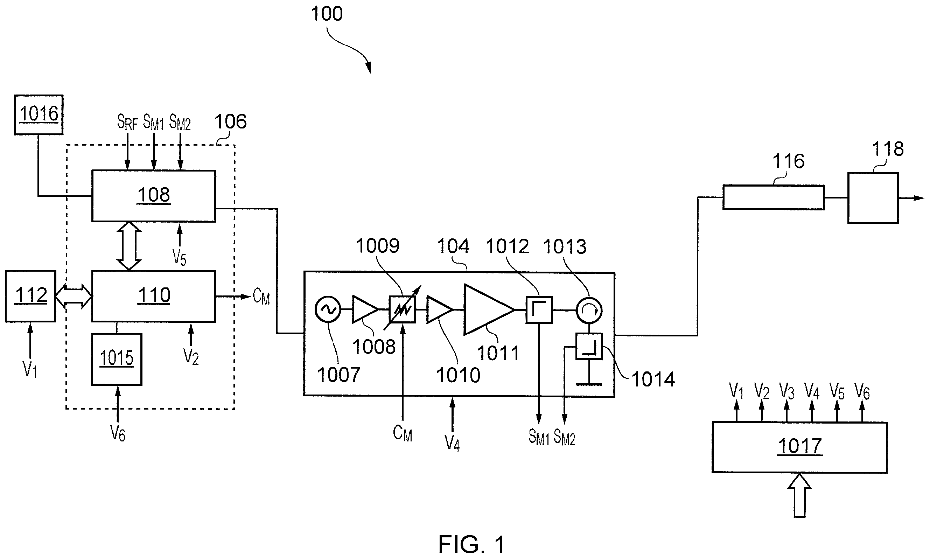

Aspects of the overall ligament treatment system and electrosurgical generator that can be used in that system are described below with reference to Figs. 1 to 8.

Fig. 1 shows an overall apparatus diagram for an electrosurgical ligament tightening apparatus 100 that is a first embodiment of the invention.

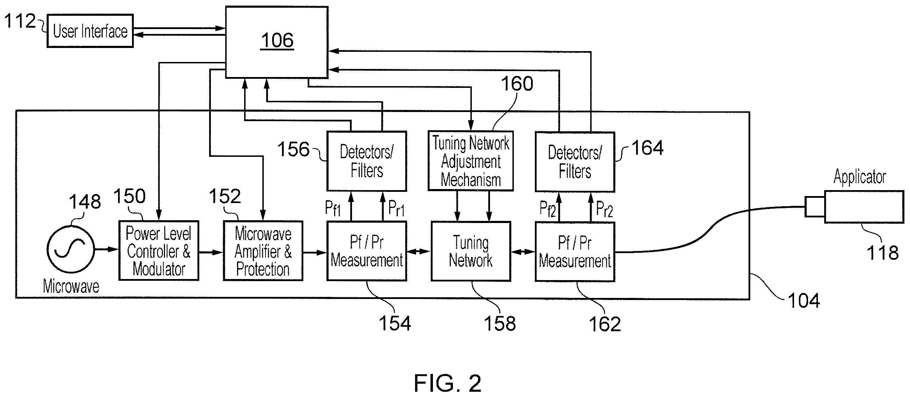

The apparatus 100 contains components for generating and controlling a microwave frequency electromagnetic signal at a power level suitable for treating (e.g. tightening) ligaments. In this embodiment the apparatus 100 includes a phase locked oscillator (microwave power source) 1007, a signal amplifier 1008, a variable signal attenuator (e.g. an analogue or digital diode attenuator) 1009, an amplifier unit (here a driver amplifier 1010 and a power amplifier 1011), a forward power coupler 1012, a circulator 1013 and a reflected power coupler 1014. The circulator 1013 isolates the forward signal from the reflected signal to reduce the unwanted signal components present at the couplers 1012, 1014, i.e. it increases the directivity of the couplers. Couplers 1012, 1014 may collectively be considered as a detector of forward and reflected signals in the generator. Optionally, the generator 104 includes an impedance matching sub-apparatus (not shown) having an adjustable impedance. This option is discussed below in more detail with reference to Fig. 2.

In this context, microwave energy is anything beyond 300 MHz, i.e. 1 GHz to 300 GHz, and preferably 2.45 GHz, 5.8 GHz, 24 GHz, etc.

The apparatus 100 includes a generator 104 in communication with a controller 106, which may comprise signal conditioning and general interface circuits 108, a microcontroller 110, and watchdog 1015. The watchdog 1015 may monitor a range of potential error conditions, which could result in the apparatus not performing to its intended specification, i.e. the apparatus delivers the wrong dosage of energy into patient body tissue due to the output or the treatment time being greater than that demanded by the user. The watchdog 1015 comprises a microprocessor that is independent of the microcontroller 110 to ensure that the microcontroller 110 is functioning correctly. The watchdog 1015 may, for example, monitor the voltage levels from DC power supplies or the timing of pulses determined by the microcontroller 110. The controller 106 is arranged to communicate control signals to the components in the generator 104. In this embodiment, the microprocessor 110 is programmed to output a microwave control signal $C_M$ for the variable signal attenuator 1009. This control signal is used to set the energy delivery profile and the power magnitude thereof to be delivered by the antenna of the microwave EM radiation output from generator 104. In particular, the variable signal attenuator 1009 is capable of controlling the power level of the output radiation. For example, the attenuator is preferably arranged to maintain a measurement mode with a 10mW measurement power magnitude until a region with no nerve tissue is detected, at which point the controller 106 controls the attenuator to switch the apparatus/generator output to the therapeutic mode, with an increased power magnitude. Moreover, the adjustable signal attenuator 1009 may include switching circuitry capable of setting the waveform (e.g. pulse width, duty cycle, etc.) of the output radiation.

The microprocessor 110 is programmed to output the microwave control signal $C_M$ based on signal information from the forward and reflected power couplers 1012, 1014. In this embodiment, the microwave generator may be controlled by measurement of phase information only, which can be obtained from the generator (from sampled forward and reflected power information). The forward power coupler 1012 outputs a signal $S_{M1}$ indicative of the forward power level and the reflected power coupler 1014 outputs a signal $S_{M2}$ indicative of the reflected power level. The signals $S_{M1}$, $S_{M2}$ from the forward and reflected power couplers 1012, 1014 are communicated to the signal conditioning and general interface circuits 108, where they are adapted to a form suitable for passing to the microprocessor 110.

A user interface 112, e.g. touch screen panel, keyboard, LED/LCD display, membrane keypad, footswitch or the like, communicates with the controller 106 to provide information about treatment to the user (e.g. clinician/surgeon) and permit various aspects of treatment (e.g. type of ligament tissue to be treated) to be manually selected or controlled, e.g. via suitable user commands. The apparatus may be operated using a conventional footswitch 1016, which is also connected to the controller 106.

The controller 106 comprises a memory (not shown) and is arranged to execute software instructions to operate the apparatus. In particular, the controller 106 controls the magnitude and profile (i.e. pulse shape and duration) of the forward power signal supplied to the probe. This control may be based on a change in the obtained information indicative of tissue type at the distal end of the antenna as the antenna is moved relative to the patient, or based on a comparison of the obtained information with predetermined reference data, which may be stored in the memory. For example, the memory may store threshold conditions for impedance measurements, whereby obtained information that satisfies a threshold condition (i.e. a threshold condition indicative of the presence of ligament tissue, and/or indicative of the absence of nerve tissue) may trigger treatment.

The apparatus may thus permit the amount of microwave power (i.e. tissue heating dosage) delivered into the treatment zone to be set up by the clinician, and may provide dynamic control over the power being delivered by continuously sampling forward and reflected power levels and making adjustments to ensure that the delivered power is the same as the demand. The user interface 112 in communication with the controller 106 allows a user (e.g. clinician or surgeon) to enter a set of user defined parameters and also display useful information, e.g. selected energy dosage and delivered energy into tissue. The user interface 112 may also enable engineering parameters to be displayed, for example reflected and forward power as a function of time. This information can be used to establish optimal energy profiles.

The control software may run on a single board computer, e.g. a microprocessor board or a DSP. The user interface 112 may comprise of a suitable flat screen display and a membrane keypad, or a touch screen display.

The apparatus may be controllable by a footswitch (not shown) or a switch in a hand piece containing the antenna 118.

Finally, the apparatus includes a power supply unit 1017 which receives power from an external source 1018 (e.g. mains power) and transforms it into DC power supply signals $V_1$-$V_6$ for the components in the apparatus. Thus, the user interface receives a power signal $V_1$, the microprocessor 110 receives a power signal $V_2$, the generator receives a power signal $V_4$, the signal conditioning and general interface circuits 108 receives a power signal $V_5$, and the watchdog 1015 receives a power signal $V_6$.

Fig. 2 is an apparatus diagram of an electrosurgical apparatus 101 according to a second embodiment of the invention. The sub-components of a generator section 104 of the apparatus are illustrated, and in this embodiment includes tuning elements, as explained below. Components in common Fig. 1 are given the same reference numbers and are not described again.

The generator 104 includes a microwave power source 148 that is used to generate low power microwave energy. The power source 148 may be a voltage controlled oscillator (VCO), dielectric resonator oscillators (DRO), Gunn diode oscillator or the like. The output of the power source 148 is received by a power level controller and modulator unit 150. The power level controller and modulator unit 150 may include a signal modulation device arranged to enable the generator to be operated in a pulsed mode, and a power control attenuator arranged to enable the user to control the level of power delivered into the tissue. For ligament treatment a single pulse of energy, e.g. 50 W for 20 ms may be sufficient to heat the ligament in order to tighten it. The signal modulation device provides the ability to control the pulse duration.

The attenuator in the modulation unit is used to enable the user to control the level/magnitude of power delivered into the tissue, e.g. ligament tissue in the treatment zone. The output of the modulation switch is input to an amplifier and protection unit 152 which is arranged to amplify the power signal to a power level suitable for treatment, i.e. suitable for causing the very quick temperature rise in biological tissue in the treatment zone in order to cause ligament tightening. The first power level may be 10 W or more, e.g. 50 W. The attenuator can be used to control the input power, and hence indirectly the output power, of the amplifier 152. Alternatively, the attenuator may be omitted and a control signal may be used to control the power level, e.g. by controlling the gain of amplifier and protection unit 152.

The amplifier and protection unit 152 may include a driver amplifier to amplify the output signal level produced by the frequency source 148, and a power amplifier to amplify the signal produced by the driver amplifier to a level suitable to cause ligament tightening. Hence, the amplifier and protection unit 152 may be controlled by the controller 106 to switch the generator output from the measurement mode to the therapeutic mode (e.g. by amplifying the forward power signal to the therapeutic power magnitude) when the measurement mode detects that there is no nerve tissue present in the treatment zone. To protect the amplifiers and source from high levels of reflected microwave energy, the output from the power amplifier may be connected to a microwave circulator. The circulator only allows microwave power to flow in a clockwise direction, hence any reflected power coming back into power amplifier will be absorbed by a power dump load if the circulator is a three port device, where the first port takes in the output power from the amplifier. The second port outputs this power into a feed structure and probe and receives power back from the probe and feed structure when the distal end of the probe is mismatched with the impedance of the body tissue. The third port is connected to a power load that is capable of absorbing the reflected power and is very well matched with the impedance of the circulator. The impedance of the matched load is preferably the same as the impedance of the apparatus, i.e. 50 + j0 Ω. A directional coupler may be connected between the third port of the circulator and the input to the matched load to enable the reflected power to be sampled.

The output of the amplifier and protection unit 152 is input to a first power coupling unit 154, which may comprise a forward directional coupler and reflected directional coupler arranged to sample the forward and reflected microwave energy on the generator. The sampled forward and reflected power levels are input respectively to a forward and reflected first power detection unit 156, in which the power levels are detected, e.g. using diode detectors or heterodyne/homodyne detectors, to sample a portion of the forward and reflected power and enable magnitude, or magnitude and phase, or phase only information to be extracted from the sampled signal. The signals produced by the first power detection unit 156 are input to the controller 106 to enable magnitude and/or phase of forward and reflected power to be used to calculate the net power delivered into the tissue and to determine the necessary input signals going into the power level controller and modulator 150 to ensure that the actual delivered power or energy is equal to the demanded power or energy.

The magnitude of the forward and return signals (indicative of the attenuation of the body tissue in the treatment zone), an indicator of the dielectric properties of the tissue in the treatment zone, can then be used to determine presence/absence of nerve tissue and/or ligament tissue. The forward power can then be adjusted accordingly. Additionally, or alternatively, phase information from the forward and return signals can be used. As discussed above, the phase and/or attenuation information may be compared with predetermined reference data to determine the type of tissue in the treatment zone.

This embodiment may also use a dynamic impedance matching apparatus (impedance adjuster) to enable the microwave energy developed by the amplifier and protection unit 152 to be matched, in terms of impedance, with the load presented to the distal end of the probe 118 by the tissue in the treatment zone, when it is determined (from the measured dielectric properties) that the treatment zone does not contain nerve tissue. This invention is not limited to the use of an automatic tuning mechanism for the microwave power delivery apparatus, i.e. the distal end of the probe (the radiator) may be matched to one particular biological tissue type/state at the frequency of operation or the impedance of the probe may be mechanically adjusted, i.e. by a mechanism included in the hand-piece to provide a level of matching between the probe impedance and the impedance of the tissue in contact with the probe. The output of the first power coupling unit 154 is received by a tuning network 158, which has an adjustable impedance on the generator 104 that is determined by the state of a tuning network adjustment mechanism 160 under the control of controller 106, based on information gathered from first power detection unit 156 and a second power detection unit 164.

The output of the impedance adjuster 158 is input to a second power coupling unit 162, which may be configured in a similar manner to the first power coupling unit 154 to sample forward and reflected power levels from the generator 104 and input them respectively to a second forward and reflected power detection unit 164, which forwards the detected power levels and/or phase information to the controller 106.

The information made available by the first and second power detection units, 156, 164 may be compared to determine the adjustments required to the impedance adjuster 158 to enable the power source to be impedance matched to the impedance of body tissue in the treatment zone.

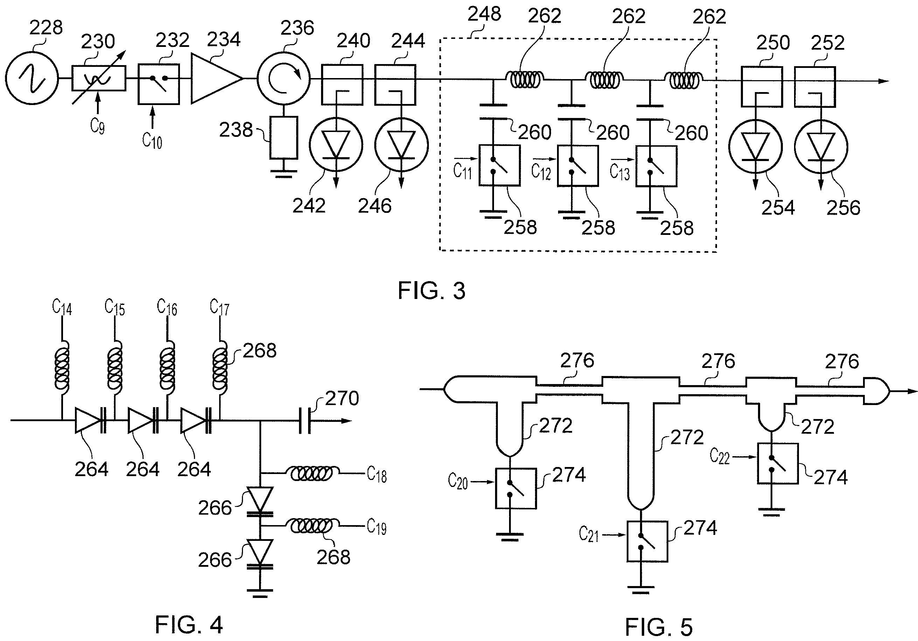

More detailed examples of the generator 104 are discussed below with reference to Figs. 3 to 5.

In use, the controller 106 operates to control the values of capacitance and inductance of the distributed tuning elements of the impedance adjuster 158 during the supply of microwave energy to match the impedance of the respective channels to the load at the distal end of the probe 118. In practice, the tuning elements of the impedance adjuster may be variable stubs/microstrip transmission lines or power PIN/Varactor diodes (distributed elements). Impedance matching in this context refers to maximising the transfer of energy into tissue (through radiation of microwave energy) by complex conjugate matching of the source (i.e. the apparatus) to the tissue in the treatment zone. It may be noted that the microwave source can deliver energy by radiation and conduction, but the return path is localised for the microwave currents.

It may be preferable for oscillator 148 to be phase locked to a stable temperature compensated crystal reference source in order for energy at the microwave frequency to be at a fixed, temperature-stable frequency.

The impedance adjuster may be used to ensure that the antenna structure in contact with tissue is well matched to the impedance of the tissue to ensure maximum energy transfer to ligament (non-nerve) tissue is achieved and that the energy delivered from the radiating section of the applicator can be well quantified, i.e. taking into account the insertion loss of the delivery cable and the applicator, a user demand of 10 W for 10 seconds to deliver 100 J of energy into the target tissue can be achieved with a high degree of confidence even when the impedance of the tissue (i.e. collagen) changes as a result of heating effects.

Fig. 3 shows a schematic drawing of the components of the generator of the apparatus to an embodiment. The power source 228 outputs a microwave signal having a stable (e.g. fixed) microwave frequency. The output from the power source 228 is input to a variable attenuator 230, which controls the magnitude of the output based on a control signal Cg from the controller (not shown). The output from the variable attenuator 230 is input to a switch unit 232, which modulates the output based on a control signal $C_{10}$ from the controller. In practice, units 230 and 232 could be combined into one single unit by using a variable attenuator with a response time (time to change the signal attenuation when in receipt of the new digital input signals) that is fast enough to allow the device to act as a modulator or to allow the apparatus to operate in pulsed mode, i.e. if the response time of the attenuator is 100 ns and the apparatus is to be operated in pulsed mode, where the width of the pulse is required to be 5 ms and the off time between pulses is 20 ms, then this device can quite easily be used to serve two purposes. The output of the switch unit 232 is received by a power amplifier 234, which amplifies the microwave signal to a power level suitable to produce a useful therapeutic ligament tightening effect when no nerve tissue is detected in the treatment zone. The output from the power amplifier 234 is input to the first port of a circulator 236. The circulator 236 isolates the amplifier from reflected signals travelling back from the probe. Any reflected signal received back at the second port of the circulator is directed out of the third port into a power dump load 238.

The forward signal from the amplifier is output from the second port of the circulator, which is connected to a forward directional coupler 240, which couples a portion of the forward directed signal into a detector 242. The output of the detector 242 is connected to the controller. The output of the forward directional coupler 240 is input to a reverse directional coupler 244, which couples a portion of any reflected signal into a detector 246. The output of the detector 246 is connected to the controller. The output of the reverse directional coupler 244 is input to a microwave impedance adjuster 248 that has an adjustable impedance. The output of the impedance adjuster 248 is input to a forward directional coupler 250 and reverse directional coupler 252 for coupling a portion of the forward and reflected signal respectively into detectors 254, 256 in a manner similar to the forward and reverse directional couplers 240, 244. The outputs of the detectors 254, 256 are connected to the controller. This invention is not limited to the use of diode detectors, i.e. log magnitude detectors, homodyne phase and magnitude detectors, heterodyne phase and magnitude detectors or Exclusive OR gate (XOR) phase detectors may be used to implement 242, 246, 254 and 256. The ability to extract phase information as well as magnitude information is beneficial in terms of being able to make accurate and dynamic adjustments of the microwave tuning network, provide a greater degree of control, effectively prevent nerve damage, and improve the performance of the matching apparatus in terms of accessible impedances that can be matched to, but the invention is not limited by the need to extract phase as well as magnitude information to control the apparatus. The measurement information in the generator may be made by measuring phase information only, for example.

The controller may use the outputs from the diode detectors (or other types of detectors) 242, 246, 254, 256 to determine the amount of power delivered to the load (e.g. ligament tissue) and/or as a means for controlling the impedance of the impedance adjuster 248 to minimise the reflected power and match the energy produced by the generator into the changing impedance of the tissue load to provide optimal efficiency of energy delivery into ligament tissue as its dielectric properties change through heating, and to provide optimal apparatus performance in terms of minimisation of component heating due to energy being returned to the generator and accurate quantification of energy delivery into target ligament (non-nerve) tissue.

The impedance adjuster 248 in Fig. 3 comprises three PIN diode switches 258 connected in shunt to the generator. Each PIN diode switch 258 has an independent DC or relatively low frequency, i.e. up to 10 kHz, voltage control signal $C_{11}$-$C_{13}$ (produced by the controller) for controlling its status. The PIN diode switches operate to switch a respective shunt capacitance 260 (which may be formed by a section of transmission line, i.e. microstrip or co-axial) into the generator. Series inductors 262 (which may also be a section of transmission line) are shown connected between the shunt elements. The combination of shunt capacitance and series inductance form a tuning network or filter and the ability to switch individual elements that form the overall value of capacitance or inductance in and out allows the network to act as a variable tuning filter. In order to increase the tuning range, the number of elements in the network may be increased. The fixed values of shunt capacitance that make up the overall value of tuning capacitance may be weighted, i.e. binary weighted, to provide as large as possible range of variation. The position of the inductors and capacitors that form the impedance adjuster/tuning network may be interchanged, i.e. the inductors may be connected in shunt and the capacitors in series. Values of capacitance and inductance used in the network may be realised by inserting transmission lines of varied length between the shunt elements and/or between the transmission lines and the switches connected in shunt across the tuning element, i.e. a length of transmission line of physical length equal to one eighth of the guided wavelength will produce an inductive reactance of value equal to the characteristic impedance of the transmission line.

The impedance adjuster 248 may be implemented in other ways. Fig. 4 shows an alternative arrangement in which a plurality of first varactor diodes (or power PIN diodes) 264 are connected in series on the generator and a plurality of second varactor diodes (or power PIN diodes) 266 are connected in parallel to the generator. Controllable DC bias signals $C_{14}$-$C_{19}$ can be applied to control the voltage across each varactor diode 264, 266 to modify the length of the depletion region, which in turn varies the capacitance. Blocking inductors 268 prevent microwave energy from going back into the DC source. These inductors may be realised in microstrip, i.e. a printed inductor or small coils of wire. In this manner the series varactor diodes act as a part of a transmission line having an electrical length that can be varied by up to $\dfrac{\lambda}{2}$, where $\lambda$ is the wavelength of the microwave energy. The parallel shunt varactor diodes may act as a stub having an electrical length that can be varied by up to $\dfrac{\lambda}{4}$. A DC blocking capacitor 270 is connected between the tuning network and the probe to prevent DC or low frequency AC currents from being delivered into the patient, i.e. it provides a DC patient isolation barrier.

Fig. 5 shows another alternative arrangement for the impedance adjuster, implemented using microstrip stubs. In this example, three microstrip stubs 272 having differing lengths are connected to a microstrip line on the generator. Each stub 272 can be independently switched between short circuit (switch contact or junction closed) and open circuit (switch or channel open) using PIN diode (or electromechanical) switches 274 under the control of DC signals $C_{20}$-$C_{22}$. The transmission line that forms the stub 272 can be set to a length that represents a range of reactances (capacitive or inductive) or impedances. The arrangement shown in Fig. 5 enables eight different tuning positions, i.e. $2^3$, to be selected. As in the Fig. 3 example, inductors 276 are shown connected in series between the shunt stubs. These inductors are shown here as thin transmission lines realised in microstrip line by printing lines onto a dielectric material that are narrower than the lines that form the characteristic impedance of the transmission line. Other transmission line configurations, where the width/diameter and/or length of the line enables inductors of required inductance at the frequency of operation to be realised, may also be used. This configuration is not limited to using inductors 276, i.e. the width of the microstrip line may be increased to be greater than that required to form a line with impedance equal to the characteristic impedance of the transmission line in order to produce a tuning capacitance rather than a tuning inductance.

In another example, transmission line stubs or waveguide (rectangular or cylindrical) sections that form the stubs may be used instead of microstrip stubs and co-axial trombone structures may be implemented to vary phase.

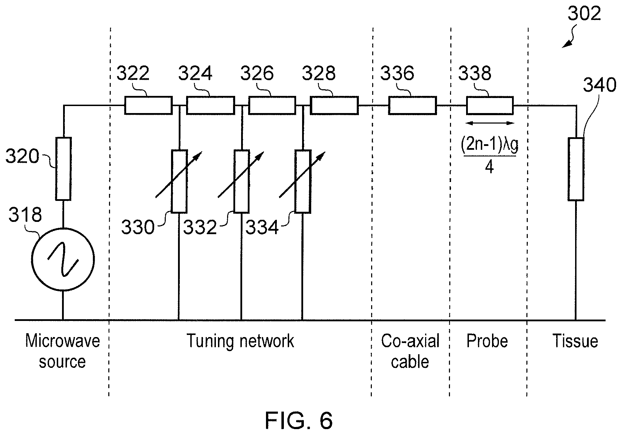

Fig. 6 shows a distributed circuit 302 for the generator that may be used to analyse the operation of the electrosurgical apparatus.

The analysis of the generator shown in Fig. 6 is based on a distributed network of impedances, where each element is represented as a complex impedance. Microwave generator 318 is shown connected in series to the impedance of the generator 320 and is nominally $50\,\Omega$. The source impedance is connected to a distributed element microwave tuner comprising of four series connected fixed impedances 322, 324, 326, 328 and three shunt connected variable impedances 330, 332, 334 connected between the distal and proximal ends of the aforementioned series impedances. The output of the tuning network is connected to the co-axial cable assembly, which has a nominal impedance 336 of $50\,\Omega$.

From the distributed element microwave tuning apparatus represented by a range of impedance values and variable/fixed line lengths and shown in Fig. 6, the variable elements 330, 332, 334 within the tuning network must match the source impedance 320 to the tissue impedance 340 when the co-axial cable assembly (with impedance 336) and antenna (with impedance 338) are connected between the output port of the impedance tuner and the tissue in contact with the antenna.

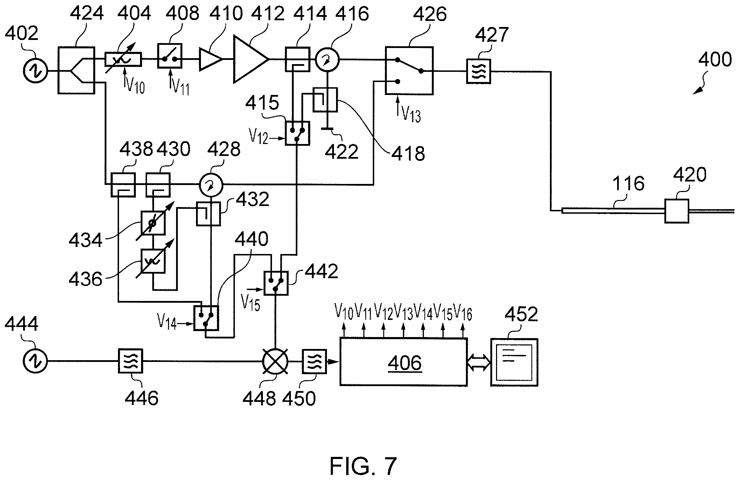

Fig. 7 shows a complete apparatus diagram for electrosurgical apparatus 400 according to a third embodiment of the invention. In this embodiment, the generator has a microwave power source 402, a therapeutic channel, and a measurement channel separate from the therapeutic channel.

The therapeutic channel comprises a power control module comprising a variable attenuator 404 controlled by controller 406 via control signal $V_{10}$ and a signal modulator 408 controlled by controller 406 via control signal $V_{11}$, and an amplifier module comprising drive amplifier 410 and power amplifier 412 for generating forward microwave EM radiation for delivery from a probe 420 at a power level suitable for treatment. After the amplifier module, the therapeutic channel continues with a microwave signal coupling module (which is part of the microwave signal detector) comprising a circulator 416 connected to deliver microwave EM energy from the source to the probe along a path between its first and second ports, a forward coupler 414 at the first port of the circulator 416, and a reflected coupler 418 at the third port of the circulator 416. After passing through the reflected coupler, the microwave EM energy from the third port is absorbed in a power dump load 422. The microwave signal coupling module also includes a switch 415 operated by the controller 406 via control signal $V_{12}$ for connecting either the forward coupled signal or the reflected coupled signal to a heterodyne receiver for detection.

To create the measurement channel in this embodiment, a power splitter 424 (e.g. a 3 dB power splitter) is used to divide the signal from the source 402 into two branches. In an alternative embodiment, the power splitter 424 may be omitted and a separate source used for the measurement channel. One branch from the power splitter 424 forms the therapeutic channel, and has the components described above connected thereon. The other branch forms the measurement channel. The measurement channel bypasses the amplifier(s) on the therapeutic channel, and hence is arranged to deliver a low power signal from the probe, e.g. a 10mW CW power signal suitable for use in the measurement mode to detect the type of tissue in the treatment zone, without causing heating significant effects in the treatment zone. In this embodiment, a primary channel selection switch 426 controlled by the controller 406 via control signal $V_{13}$ is operable to select a signal from either the therapeutic channel or the measurement channel to deliver to the probe. For example, the controller 406 may cause the switch 426 to switch to the therapeutic channel for delivering a high power output to perform ligament tightening, when it is determined in the measurement mode that no nerve tissue is present in the treatment zone.

The measurement channel in this embodiment includes components arranged to detect the phase and magnitude of power reflected from the probe, which may yield information about the material e.g. type (ligament or nerve) of biological tissue present at the distal end of the probe. The measurement channel comprises a circulator 428 connected to deliver microwave EM energy from the source 402 to the probe along a path between its first and second ports. A reflected signal returned from the probe is directed into the third port of the circulator 428. The circulator 428 is used to provide isolation between the forward signal and the reflected signal to facilitate accurate measurement. However, as the circulator does not provide complete isolation between its first and third ports, i.e. some of the forward signal may break through to the third port and interfere with the reflected signal, a carrier cancellation circuit is used that injects a portion of the forward signal (from forward coupler 430) back into the signal coming out of the third port (via injection coupler 432). The carrier cancellation circuit include a phase adjustor 434 to ensure that the injected portion is 180° out of phase with any signal that breaks through into the third port from the first port in order to cancel it out. The carrier cancellation circuit also include a signal attenuator 436 to ensure that the magnitude of the injected portion is the same as any breakthrough signal.

To compensate for any drift in the forward signal, a forward coupler 438 is provided on the measurement channel. The coupled output of the forward coupler 438 and the reflected signal from the third port of the circulator 428 are connected to respective input terminal of a switch 440, which is operated by the controller 406 via control signal $V_{14}$ to connect either the coupled forward signal or the reflected signal to a heterodyne receiver for detection.

The output of the switch 440 (i.e. the output from the measurement channel) and the output of the switch 415 (i.e. the output from the therapeutic channel) are connected to a respective input terminal of a secondary channel selection switch 442, which is operable by the controller 406 via control signal $V_{15}$ in conjunction with the primary channel selection switch to ensure that the output of the measurement channel is connected to the heterodyne receiver when the measurement channel is supplying energy to the probe and that the output of the therapeutic channel is connected to the heterodyne receiver when the therapeutic channel is supplying energy to the probe.

The heterodyne receiver is used to extract the phase and magnitude information from the signal output by the secondary channel selection switch 442. In the embodiment shown in Fig. 7 a single heterodyne receiver is used. A double heterodyne receiver (containing two local oscillators and mixers) to mix the source frequency down twice before the signal enters the controller may be used if necessary. The heterodyne receiver comprises a local oscillator 444 and a mixer 448 for mixing down the signal output by the secondary channel selection switch 442. The frequency of the local oscillator signal is selected so that the output from the mixer 448 is at an intermediate frequency suitable to be received in the controller 406. Band pass filters 446, 450 are provided to protect the local oscillator 444 and the controller 406 from the high frequency microwave signals.

The controller 406 receives the output of the heterodyne receiver and determines (e.g. extracts) from it information indicative of phase and magnitude of the forward and/or reflected signals on the therapeutic and/or measurement channel. This information can be used to control the delivery of high power microwave EM radiation on the therapeutic channel, e.g. depending on the type of ligament tissue detected in the treatment zone. In an embodiment, the controller switches the apparatus to deliver high power microwave EM radiation when the dielectric properties of the material in the treatment zone, as determined from the forward and reflected signals, are indicative of a treatment zone containing no nerve tissue. As discussed above, this determination may be made with the use of reference data. A user may also interact with the controller 406 via a user interface 452, as also discussed in the above embodiments.

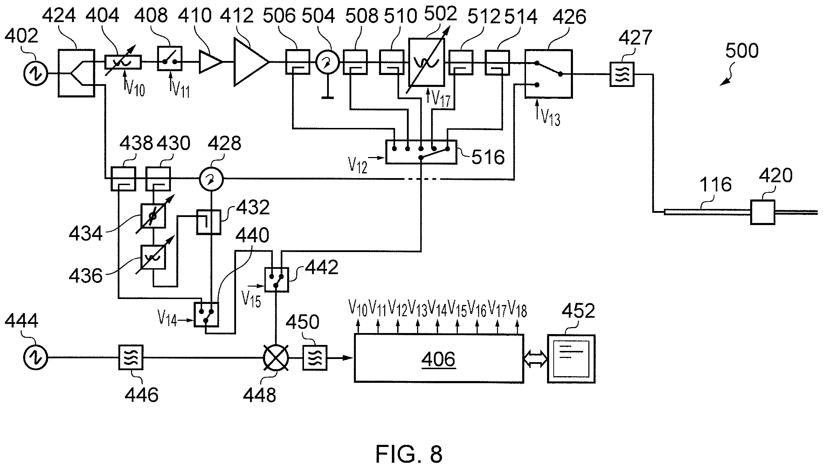

Fig. 8 shows a complete apparatus diagram for electrosurgical apparatus 500 that is a slight modification of the apparatus shown in the third embodiment of Fig. 7. Components in common between Figs. 7 and 8 are given the same reference number and are not described again.

On the therapeutic channel an impedance adjuster 502 is connected between the amplifier module and probe. The impedance adjuster 502 is controlled by controller 406 via control signal $V_{17}$. A circulator 504 acts as an isolator between the amplifier module and impedance adjuster 502 to protect the power amplifier 412 from reflected signals. A forward coupler 506 connected between the power amplifier 412 and circulator 504 couples out a power amplifier monitoring signal. A forward coupler 508 and reflected coupler 510 are connected between the circulator 504 and impedance adjuster 502 to provide information about forward and reflected power signals on the generator before the impedance adjuster 502. A forward coupler 512 and reflected coupler 514 are connected between impedance adjuster 502 and probe 420 to provide information about forward and reflected power signals on the generator after the impedance adjuster 502. In combination, the couplers 508, 510, 512, 514 can extract information that permits the controller 406 to determine the power delivered from the probe and the power loss in the impedance adjustor 502. The latter is optional, so only one pair of couplers 512, 514 may be needed. A signal selection switch 516 operable by the controller 406 via control signal $V_{12}$ connects one of the outputs of the couplers 506, 508, 510, 512, 514 to the heterodyne receiver from where it is sent to the controller 406 to provide the microwave signal information.

Phase and magnitude information available can be used to control the variable elements contained within the impedance adjuster 502 to maximise the efficiency of energy delivery from the therapeutic channel.

*Probe structures*

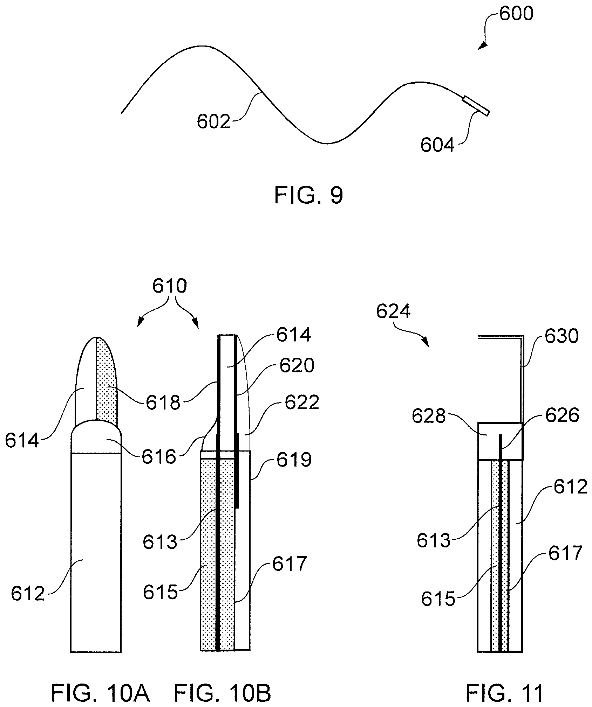

Probe structures suitable for use with the apparatus discussed above are now described with reference to Figs. 9 to 16. A general probe structure 600 is shown in Fig. 9. The probe comprises a flexible shaft 602 that contains (e.g. conveying through a lumen thereof) a microwave cable (e.g. a coaxial cable) that can be positioned at a target site. At a distal end of the flexible shaft 602 there is an applicator 604 that has an energy delivery structure connected to receive microwave electromagnetic (EM) energy from the cable and to deliver that energy to tissue at the target site. Example configurations for the energy delivery structure are discussed below.

The energy may be delivered in a directional manner, e.g. to give the operator control over the region of tissue to be treated through appropriate orientation of the applicator. A proximal end of the flexible shaft 602 may be connected to a generator (not shown in Fig. 9) which supplies and controls the microwave EM energy as discussed above.

Two use scenarios are envisaged. If the probe is used in open or general surgery, one or more guide wires (not shown) may be conveyed through a lumen in the shaft 602. The applicator 604 may comprise a flexible tip which can be moved by manipulating the guide wires. If the probe is used with a surgical scoping device, e.g. with an endoscope in anterior cruciate ligament surgery or Achilles tendon reconstruction, the applicator 604 and flexible shaft 602 may be inserted through the instrument channel of the scoping device. In this example, movement (e.g. steering) of the probe may be controlled by manipulating the endoscope.

Figs. 10A and 10B show a first example probe 610. The probe 610 comprises an energy delivery structure mounted at a distal end of a shaft 612. In this example, the energy delivery structure comprises a planar body 614 of dielectric material (e.g. ceramic or the like) having a curved distal edge (e.g. substantially in the shape of a parabola). A top surface of the planar body 614 has a first conductive material 618 formed (e.g. deposited) on one side thereof. The conductive material may be metal, e.g. gold or stainless steel. Similarly, a second conductive material 620 may be formed on a bottom surface of the planar body 614. The bottom surface has a protective hull 622 mounted over it. The protective hull 622 is made of a dielectric material and tapers gradually to the edge of the planar body 614.

As shown in the cross-sectional side view of Fig. 10B, a coaxial cable is conveying within the shaft 612. The coaxial cable comprises an inner conductor 613, an outer conductor 617 and a dielectric material 615. The inner conductor 613 extends distally beyond a distal end of the dielectric material 615 to electrically contact the first conductive material 618. The outer conductor 617 is electrically connected to the second conductive material 620 by a conductive link 619. In this manner the first conductive material 618 and the second conductive material 620 form an energy delivery structure. When microwave energy is delivered to the probe, microwave energy radiates out from the side of the body 614 that is covered with the conductive material 618.

A tapering shield cover 616 is mounted over the connection between the inner conductor 613 and first conductive material to protect the junction.

Provides the conductive coating on only part of the top surface of the body 614 gives the probe 610 directionality in the manner in which it radiates energy.

Fig. 11 shows a second example probe 624. Features in common with Figs. 10A and 10B are given the same reference number and are not described again. In this example, the applicator comprises a simple microwave antenna formed by mounted a dielectric cap 628 on a distal end of the shaft 612. The inner conductor 613 of the coaxial cable includes a distal portion 626 that protrudes beyond the rest of the coaxial cable into the dielectric cap to form the antenna. Dielectric properties of the dielectric cap 628 are chosen to provide a desirable field shape. The probe 624 further comprises a hook element 630 that extends beyond a distal end of the dielectric cap 628. The hook 630 can be used to grapple the target tissue before the microwave energy is applied. The hook 630 may be retractable, e.g. by manipulation of a suitable guide rod (not shown).

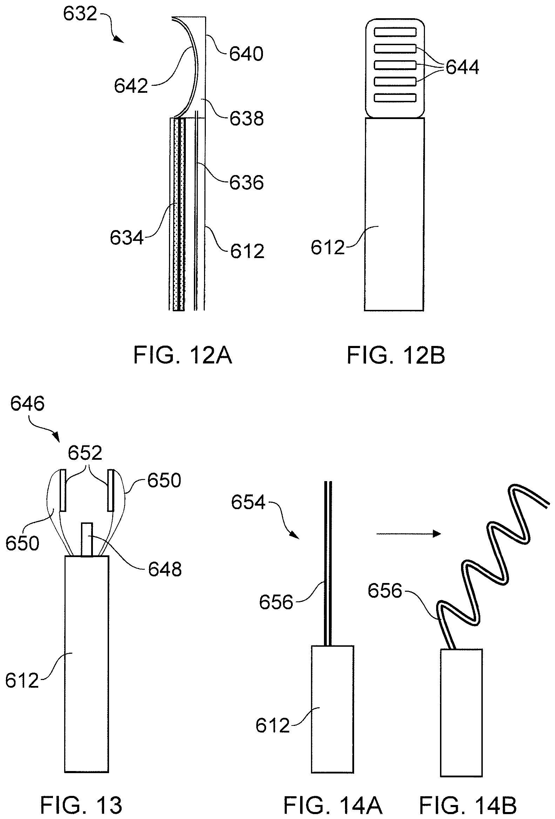

Figs. 12A and 12B shows a third example probe 632. Features in common with Figs. 10A and 10B are given the same reference number and are not described again. The energy delivery structure used by the probe 632 is a "leaky feeder" type transmission line, where slots are formed in a ground plane to permit energy to escape. In this example, the energy delivery structure comprise a flexible dielectric sheet 642 that is metallised on both sides. An outer metallisation layer is connected to an outer conductor of a coaxial cable 634 conveyed through the shaft 612. An inner metallisation layer is connected to an inner conductor of the coaxial cable 634. As shown in Fig. 12B, a plurality of slots 644 are formed in the outer metallisation layer to formed a travelling wave slotted antenna. The size and position of the slots is selected based on the properties of the flexible dielectric sheet 642 and the frequency of the microwave energy in a known manner. In this example, the probe 632 is further configured to permit the flexible dielectric sheet to expand against and conform to (e.g. wrap around) tissue at the target site. This is done by mounting the flexible sheet within a frame 640 and providing an inflatable volume 638 between the frame 640 and flexible sheet 642. The inflatable volume 638 (which may be a balloon or similar) is in fluid communication with an inflation medium (e.g. a suitable inert or biocompatible gas or liquid) to permit controllable inflation thereof. A fluid supply conduit 636 may be conveyed through the shaft 612 for this purpose. The inflatable volume may have a predetermined shape arranged so that, when inflated, it causes the flexible sheet 642 to exhibit a desired shape. For example, it may be desirable for the flexible sheet to present a concave surface to tissue at the target site.

Fig. 13 shows a fourth example probe 646. Features in common with Figs. 10A and 10B are given the same reference number and are not described again. The applicator of probe 646 is in the form of a grasper defined by a pair of jaws 650. The jaws 650 are disposed in an opposed manner to define a space therebetween for receiving tissue to be treated. One or both jaws 650 may have an energy delivery structure 652 mounted thereon to contact tissue present in the space. The jaws 650 may be adjustable to open and close the space. In this embodiment there is also a transducer 648 arranged to detect information indicative or temperature in the space. The transducer 648 may be a thermocouple or the like.

Any of the other probe structures discussed herein may be provided with a similar transducer. In other examples, an imaging element (e.g. optical fibre bundle with a lens) may be used with or in place of the transducer to monitor the treatment site.

Figs. 14A and 14B shows a fifth example probe 654. Features in common with Figs. 10A and 10B are given the same reference number and are not described again. In this example, the applicator comprises a wire structure 656 having an adjustable shape. As shown schematically in Fig. 14B, the wire structure 656 may be configured to adopt a helical shape when in use, e.g. to wrap around tissue to be treated. Energy may be delivered from the wire structure at the points where it contacts the tissue. The wire structure 656 may be resiliently deformable away from the helical configuration into a straighter configuration as shown in Fig. 14A. The deformation of the wire structure may be performed by manipulating one or more guide rods that extends through the shaft 612. The applicator may be positions at the target site with the wire structure 656 in the straighter configuration, whereupon it can be released to adopt the helical configuration to surround tissue to the treated.

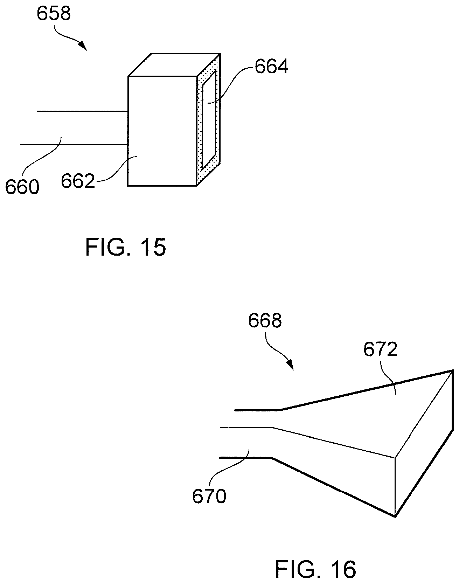

Fig. 15 shows a sixth example probe 658. In this example, the applicator is an open rectangular waveguide 662 attached at the distal end of a feed cable 660. The power delivered by an open waveguide 662 varies across the aperture of the waveguide as a cosine the squared electric field. The electric field is zero at the side walls of the aperture and a maximum at the centre. The waveguide 662 may be filled with a dielectric material 664 to reduce the size of the radiating aperture. The size reduction is proportional to the square root of the dielectric constant, so if the guide is filled with a material that has a permittivity of 25, then the size reduction with be 5. In other words, a waveguide applicator with an unloaded long wall length of 25 mm, will have a loaded wall length of 5 mm if the relative permittivity of the loading material is 25. If the short wall was 10 mm, then this will be reduced to 2 mm, hence a structure that is 10 mm x 25 mm with air becomes a 2mm x 5mm structure when filled with a material that has a permittivity of 25. Material that could be used to achieve this is ECCOSTOCK® HiK500F.

Fig. 16 shows a seventh example probe 668. In this example, the applicator is a horn antenna 672 attached at the distal end of a feed cable 670. The horn antenna 672 may be configured to produce a focussed beam width, e.g. of 18 degrees, to focus the energy into the ligament. In another example, an array of antennas, e.g. horns or other structures, may be provided in the applicator. In such an example, the directionality of the emitted energy may be adjustable by controlling properties of the antennas or the signals their each receive. For example, by controlling the phase of each antenna in an array, the beam emitted by the antennas may be arranged to converge at a point.

To maintain close control over the temperature of the target site, any of the probes discussed above may be arranged to cool tissue at the surface. That can be done by delivering coolant directly to the treatment site, e.g. via a fluid feed conduit conveyed by the shaft. Or cooling may be applied independently of the probe, e.g. via the surface of the skin adjacent to a treatment site. Cooling the surface can assist in protect the skin and other tissue structures (e.g. fascia) from thermal damage. Cooling may be performed before the microwave energy is introduced, e.g. to lower the temperature of surrounding (non-target) tissue.

*Other possible fields of use*

The discussion above presents the invention in terms of tightening ligaments. In this context, the invention may find particular use in treating ligaments in the shoulder, knee and foot. The invention may also be used to tighten or other treat tendons, e.g. the Achilles tendon, etc.

The invention may also be applicable in other fields. For example, the invention may find use in management of a prolapsed uterus, e.g. to tighten muscles and associated structures which have been stretched after childbirth. Similarly, the invention may be used to tighten the muscles or create strictures around the bladder to aid urinary incontinence.

**Claims**

1. An electrosurgical apparatus for ligament tightening, the apparatus comprising:

   an electrosurgical generator arranged to generate and output microwave electromagnetic (EM) energy;
   a probe connected to the electrosurgical generator, the probe comprising:

      a flexible shaft containing a coaxial transmission line for conveying the microwave EM energy; and
      an applicator at a distal end of the flexible shaft, the applicator having an energy delivery structure arranged to receive the microwave EM from the coaxial transmission line and emit the received microwave EM energy into a treatment zone adjacent to the applicator;

   a detector arranged to monitor a property of the treatment zone; and
   a controller arranged to control an energy delivery profile of the microwave EM energy delivered to the probe based on information obtained by the detector,
   wherein the energy delivery profile is either:

(i) a measurement energy delivery profile, or

(ii) a therapeutic energy delivery profile,

wherein a power magnitude of the therapeutic energy delivery profile is larger than a power magnitude of the measurement energy delivery profile;

wherein the detector comprises a power sensing module arranged to detect a forward power signal of the measurement energy delivery profile travelling from the electrosurgical generator to the probe and a reflected power signal reflected back from the probe,

wherein the controller is arranged to process the detected forward and reflected power signals to obtain information indicative of a type of body tissue in the treatment zone, and

wherein the controller includes a memory storing reference data and a microprocessor arranged to:

execute software commands to compare the information indicative of type of body tissue in the treatment zone with the reference data, and

detect the presence of nerve tissue in the treatment zone from the comparison; and select the therapeutic energy delivery profile when it is determined that nerve tissue is absent from the treatment zone.

2. An electrosurgical apparatus according to claim 1, wherein the detector comprises a temperature sensor.

3. An electrosurgical apparatus according to claim 1 or 2, wherein the detector comprises an imaging device.

4. An electrosurgical apparatus according to any preceding claim, wherein the controller is arranged to determine from the detected forward and reflected power signals either:

(i) complex impedance, or
(ii) attenuation and/or phase constants

of the type of body tissue in the treatment zone, the information indicative of the type of body tissue in the treatment zone being a result of determining the complex impedance or the attenuation and/or phase constants.

5. An electrosurgical apparatus according to any preceding claim comprising a cooling mechanism for removing thermal energy from the treatment zone.

6. An electrosurgical apparatus according to claim 5, wherein the probe including a fluid feed conduit extending through the flexible shaft, and wherein the cooling mechanism comprises an actuator for delivering coolant through the fluid feed conduit to the treatment zone.

7. An electrosurgical apparatus according to any preceding claim comprising a surgical scoping device having a steerable instrument cord with an instrument channel extending therethrough, wherein the probe is dimensioned to be insertable through the instrument channel to reach the treatment zone.

8. An electrosurgical apparatus according to any preceding claim, wherein the power magnitude of the measurement energy delivery profile is 10 mW or less.

9. An electrosurgical apparatus according to any preceding claim 10, wherein the power magnitude of the therapeutic energy delivery profile is equal to or less than 15 W.

10. An electrosurgical apparatus according to any preceding claim, wherein the energy delivery structure comprises a travelling wave slotted radiator.

11. An electrosurgical apparatus according to any one of claims 1 to 9, wherein the energy delivery structure comprises a microstrip antenna.

12. An electrosurgical apparatus according to any one of claims 1 to 9, wherein the energy delivery structure comprises an open waveguide.

13. An electrosurgical apparatus according to any preceding claim, wherein the energy delivery structure is arranged to conform to a treatment zone on the human or animal body.

14. An electrosurgical apparatus according to any preceding claim, wherein the applicator comprises an inflatable portion arranged to expand to extend the energy delivery structure into the treatment zone.

15. An electrosurgical apparatus according to any preceding claim, wherein the probe comprises a hook portion for retain a portion of tissue against the energy delivery structure.

**Patentansprüche**

1. Elektrochirurgische Vorrichtung zur Bänderstraffung, wobei die Vorrichtung Folgendes umfasst:

einen elektrochirurgischen Generator, der angeordnet ist, um elektromagnetische (EM-) Mikrowellenenergie zu erzeugen und auszugeben;

eine Sonde, die mit dem elektrochirurgischen Generator verbunden ist, wobei die Sonde Folgendes umfasst:

einen biegsamen Schaft, der eine Koaxialübertragungsleitung zum Übertragen der EM-Mikrowellenenergie enthält; und einen Applikator an einem distalen Ende des biegsamen Schafts, wobei der Applikator eine Energiezufuhrstruktur aufweist, die angeordnet ist, um die EM-Mikrowellenenergie von der Koaxialübertragungsleitung zu empfangen und die empfangene EM-Mikrowellenenergie in eine Behandlungszone benachbart zum Applikator abzugeben;

einen Detektor, der angeordnet ist, um eine Eigenschaft der Behandlungszone zu überwachen; und

eine Steuerung, die angeordnet ist, um ein Energiezufuhrprofil der an die Sonde zugeführten EM-Mikrowellenenergie basierend auf durch den Detektor erhaltenen Informationen zu steuern,

wobei das Energiezufuhrprofil entweder eines der folgenden ist:

(i) ein Messenergiezufuhrprofil oder
(ii) ein Therapieenergiezufuhrprofil,

wobei ein Leistungswert des Therapieenergieprofils größer ist als ein Leistungswert des Messenergiezufuhrprofils;

wobei der Detektor ein Leistungsabtastmodul umfasst, das angeordnet ist, um ein vorwärtsgerichtetes Leistungssignal des Messenergiezufuhrprofils, das von dem elektrochirurgischen Generator zu der Sonde läuft, und ein reflektiertes Leistungssignal, das von der Sonde zurückreflektiert wird, zu detektieren,

wobei die Steuerung angeordnet ist, um das detektierte vorwärtsgerichtete und das reflektierte Leistungssignal zu verarbeiten, um Informationen über einen Typ von Körpergewebe in der Behandlungszone zu erhalten und

wobei die Steuerung einen Speicher, der Referenzdaten speichert, und einen Mikroprozessor umfasst, der angeordnet ist, um:

Softwarebefehle auszuführen, um die Informationen, die einen Typ von Körpergewebe in der Behandlungszone angeben, mit den Referenzdaten zu vergleichen und das Vorhandensein von Nervengewebe in der Behandlungszone anhand des Vergleichs zu detektieren; und

das Therapieenergiezufuhrprofil auszuwählen, wenn bestimmt wird, dass kein Nervengewebe in der Behandlungszone vorhanden ist.

2. Elektrochirurgische Vorrichtung nach Anspruch 1, wobei der Detektor einen Temperatursensor umfasst.

3. Elektrochirurgische Vorrichtung nach Anspruch 1 oder 2, wobei der Detektor eine Bildgebungsvorrichtung umfasst.

4. Elektrochirurgische Vorrichtung nach einem der vorangegangenen Ansprüche, wobei die Steuerung angeordnet ist, um aus dem detektierten vorwärtsgerichteten und reflektierten Leistungssignal entweder:

(i) eine komplexe Impedanz oder
(ii) Dämpfungs- und/oder Phasenkonstanten

des Typs von Körpergewebe in der Behandlungszone zu bestimmen, wobei die Informationen, die den Typ des Körpergewebes in der Behandlungszone angeben, ein Ergebnis des Bestimmens der komplexen Impedanz oder der Dämpfungs- und/oder Phasenkonstanten sind.

5. Elektrochirurgische Vorrichtung nach einem der vorangegangenen Ansprüche, umfassend einen Kühlmechanismus zum Abführen von Wärmeenergie aus der Behandlungszone.

6. Elektrochirurgische Vorrichtung nach Anspruch 5, wobei die Sonde eine Fluidzufuhrleitung umfasst, die sich durch den biegsamen Schaft erstreckt, und wobei der Kühlmechanismus einen Aktuator zum Zuführen von Kühlmittel durch die Fluidzufuhrleitung an die Behandlungszone umfasst.

7. Elektrochirurgische Vorrichtung nach einem der vorangegangenen Ansprüche, umfassend eine chirurgische Sondierungsvorrichtung, die ein lenkbares Instrumentenkabel mit einem Instrumentenkanal aufweist, der sich durch dieses hindurch erstreckt, wobei die Sonde so bemessen ist, dass sie durch den Instrumentenkanal einführbar ist, um die Behandlungszone zu erreichen.

8. Elektrochirurgische Vorrichtung nach einem der vorangegangenen Ansprüche, wobei der Leistungswert des Messenergiezufuhrprofils 10 mW oder weniger beträgt.

9. Elektrochirurgische Vorrichtung nach dem vorangegangenen Anspruch 10, wobei der Leistungswert des Therapieenergiezufuhrprofils kleiner oder gleich 15 W ist.

10. Elektrochirurgische Vorrichtung nach einem der vorangegangenen Ansprüche, wobei die Energiezufuhrstruktur einen geschlitzten Wanderwellenstrahler umfasst.

11. Elektrochirurgische Vorrichtung nach einem der Ansprüche 1 bis 9, wobei die Energiezufuhrstruktur eine Mikrostreifenantenne umfasst.

12. Elektrochirurgische Vorrichtung nach einem der Ansprüche 1 bis 9, wobei die Energiezufuhrstruktur einen offenen Wellenleiter umfasst.

13. Elektrochirurgische Vorrichtung nach einem der vorangegangenen Ansprüche, wobei die Energiezufuhrstruktur angeordnet ist, um sich einer Behandlungszone auf dem menschlichen oder tierischen Körper anzupassen.

14. Elektrochirurgische Vorrichtung nach einem der vorangegangenen Ansprüche, wobei der Applikator einen aufblasbaren Abschnitt umfasst, der angeordnet ist, um sich auszudehnen, damit die Energiezufuhrstruktur sich in die Behandlungszone erstreckt.

15. Elektrochirurgische Vorrichtung nach einem der vorangegangenen Ansprüche, wobei die Sonde einen Hakenabschnitt zum Zurückhalten eines Gewebebereichs auf die Energiezufuhrstruktur umfasst.

**Revendications**

1. Appareil électrochirurgical destiné à resserrer les ligaments, l'appareil comprenant:

   un générateur électrochirurgical conçu pour générer et délivrer une énergie électromagnétique (EM) micro-onde ;
   une sonde connectée au générateur électrochirurgical, la sonde comprenant :

   une tige flexible contenant une ligne de transmission coaxiale pour acheminer l'énergie EM micro-onde ; et
   un applicateur à une extrémité distale de la tige flexible, l'applicateur ayant une structure d'administration d'énergie conçue pour recevoir l'énergie EM micro-onde à partir de la ligne de transmission coaxiale et émettre l'énergie EM micro-onde reçue dans une zone de traitement adjacente à l'applicateur ;

   un détecteur conçu pour surveiller une propriété de la zone de traitement ; et
   un dispositif de commande conçu pour commander un profil d'administration d'énergie de l'énergie EM micro-onde administrée à la sonde sur la base d'informations obtenues par le détecteur,
   dans lequel le profil d'administration d'énergie est :

   (i) un profil d'administration d'énergie de mesure, ou bien
   (ii) un profil d'administration d'énergie thérapeutique,

   dans lequel une intensité de puissance du profil d'administration d'énergie thérapeutique est supérieure à une intensité de puissance du profil d'administration d'énergie de mesure ;
   dans lequel le détecteur comprend un module de détection de puissance conçu pour détecter un signal de puissance vers l'avant du profil d'administration d'énergie de mesure se déplaçant du générateur électrochirurgical vers la sonde et un signal de puissance réfléchi rétroréfléchi à partir de la sonde,
   dans lequel le dispositif de commande est conçu pour traiter les signaux de puissance vers l'avant et réfléchi détectés pour obtenir des informations indicatives d'un type de tissu corporel dans la zone de traitement, et
   dans lequel le dispositif de commande inclut une mémoire stockant des données de référence et un microprocesseur conçu pour :

   exécuter des commandes logicielles pour comparer les informations indicatives de type de tissu corporel dans la zone de traitement avec les données de référence, et
   détecter la présence de tissu nerveux dans la zone de traitement à partir de la comparaison ; et
   sélectionner le profil d'administration d'énergie thérapeutique lorsqu'il est déterminé que du tissu nerveux est absent de la zone de traitement.

2. Appareil électrochirurgical selon la revendication 1, dans lequel le détecteur comprend un capteur de température.

3. Appareil électrochirurgical selon la revendication 1 ou 2, dans lequel le détecteur comprend un dispositif d'imagerie.

4. Appareil électrochirurgical selon l'une quelconque des revendications précédentes, dans lequel le dispositif de commande est conçu pour déterminer à partir des signaux de puissance vers l'avant et réfléchi :

> (i) une impédance complexe, ou bien
> (ii) des constantes d'atténuation et/ou de phase
>
> du type de tissu corporel dans la zone de traitement, les informations indicatives du type de tissu corporel dans la zone de traitement étant un résultat de la détermination de l'impédance complexe ou des constantes d'atténuation et/ou de phase.

5. Appareil électrochirurgical selon l'une quelconque des revendications précédentes comprenant un mécanisme de refroidissement pour éliminer l'énergie thermique de la zone de traitement.

6. Appareil électrochirurgical selon la revendication 5, dans lequel la sonde inclut un conduit d'alimentation en fluide s'étendant à travers la tige flexible, et dans lequel le mécanisme de refroidissement comprend un actionneur destiné à administrer du réfrigérant à travers le conduit d'alimentation en fluide à la zone de traitement.

7. Appareil électrochirurgical selon l'une quelconque des revendications précédentes comprenant un dispositif d'examen chirurgical ayant un cordon d'instrument orientable avec un canal d'instrument s'étendant à travers lui, dans lequel la sonde est dimensionnée pour pouvoir être insérée à travers le canal d'instrument pour atteindre la zone de traitement.

8. Appareil électrochirurgical selon l'une quelconque des revendications précédentes, dans lequel l'intensité de puissance du profil d'administration d'énergie de mesure est de 10 mW ou moins.

9. Appareil électrochirurgical selon l'une quelconque des revendications précédentes, dans lequel l'intensité de puissance du profil d'administration d'énergie thérapeutique est inférieure ou égale à 15 W.

10. Appareil électrochirurgical selon l'une quelconque des revendications précédentes, dans lequel la structure d'administration d'énergie comprend un élément rayonnant à fentes à onde progressive.

11. Appareil électrochirurgical selon l'une quelconque des revendications 1 à 9, dans lequel la structure d'administration d'énergie comprend une antenne microruban.

12. Appareil électrochirurgical selon l'une quelconque des revendications 1 à 9, dans lequel la structure d'administration d'énergie comprend un guide d'ondes ouvert.

13. Appareil électrochirurgical selon l'une quelconque des revendications précédentes, dans lequel la structure d'administration d'énergie est conçue pour se conformer à une zone de traitement sur le corps humain ou animal.

14. Appareil électrochirurgical selon l'une quelconque des revendications précédentes, dans lequel l'applicateur comprend une partie gonflable conçue pour se dilater pour étendre la structure d'administration d'énergie dans la zone de traitement.

15. Appareil électrochirurgical selon l'une quelconque des revendications précédentes, dans lequel la sonde comprend une partie formant crochet pour retenir une partie de tissu contre la structure d'administration d'énergie.

FIG. 1

EP 3 648 695 B1

FIG. 2

FIG. 3

FIG. 4

FIG. 5

FIG. 6

FIG. 7

FIG. 8

EP 3 648 695 B1

FIG. 9

FIG. 10A   FIG. 10B

FIG. 11

24

FIG. 12A

FIG. 12B

FIG. 13

FIG. 14A    FIG. 14B

658

664

660    662

## FIG. 15

668    672

670

## FIG. 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20020095199 A
- US 6461353 B
- US 2015045786 A1